# EUROPÄISCHE PATENTSCHRIFT

(11) **EP 1 165 480 B1**
(45) Veröffentlichungstag und Bekanntmachung des Hinweises auf die Patenterteilung: **10.09.2003**
(21) Anmeldenummer: 00917005.1
(22) Anmeldetag: 28.03.2000
(51) Int. Cl.: C07C 45/82

(54) **VERFAHREN ZUR DESTILLATIVEN AUFTRENNUNG EINES FLÜSSIGEN ROHALDEHYDGEMISCHES**
METHOD FOR SEPARATING A LIQUID CRUDE ALDEHYDE MIXTURE BY DISTILLATION
PROCEDE DE SEPARATION PAR DISTILLATION D'UN MELANGE D'ALDEHYDES BRUTS LIQUIDES

(30) Priorität: 29.03.1999 DE 19914259
(43) Veröffentlichungstag der Anmeldung: 02.01.2002
(73) Patentinhaber: BASF AKTIENGESELLSCHAFT, 67056 Ludwigshafen (DE)
(72) Erfinder: MÜLLER, Rolf, D-67125 Dannstadt-Schauernheim (DE); SCHÖNMANN, Willi, D-67117 Limburgerhof (DE)
(74) Vertreter: Kinzebach, Werner, Dr.
(86) Internationale Anmeldenummer: EP0002721
(87) Internationale Veröffentlichungsnummer: WO00058255

(56) Entgegenhaltungen:
- EP-A- 0 216 151
- EP-A- 0 484 977
- DE-A- 3 320 648
- US-A- 5 227 544

## Beschreibung

Die vorliegende Erfindung betrifft ein Verfahren zur destillativen Auftrennung eines flüssigen Rohaldehydgemisches, das im Wesentlichen 95 bis 99,9 Gew.-%, bezogen auf das Gesamtgewicht des Produktgemisches, eines geradkettigen und eines verzweigten Aldehyds enthält.

Aldehyde werden großtechnisch durch Hydroformylierung von Olefinen in Gegenwart von Cobalt- oder Rhodiumkatalysatoren hergestellt. Es ist zwar im Allgemeinen erwünscht, geradkettige Aldehyde zu erhalten; je nach den Hydroformylierungsbedingungen fallen aber Gemische aus geradkettigen und verzweigten Aldehyden an. Zusätzlich zu den Aldehyden werden bei der Hydroformylierung hochsiedende Aldehydkondensationsprodukte, wie Dimere, Trimere und Tetramere der Aldehyde sowie durch Reduktion der Aldehyde die entsprechenden Alkohole als Nebenprodukte gebildet.

Das bei der Hydroformylierung anfallende Reaktionsgemisch muss daher aufgetrennt werden. Üblicherweise erfolgt dies durch ein zweistufiges Destillationsverfahren unter Verwendung von zwei getrennten, etwa gleich dimensionierten Destillationskolonnen. Dabei wird in der ersten Kolonne der gesamte Aldehyd von den hochsiedenden Bestandteilen abgetrennt. Das Gemisch aus geradkettigem und verzweigtem Aldehyd wird dann in der zweiten Kolonne aufgetrennt.

Das Verfahren unter Verwendung von zwei separaten Kolonnen ist sehr energie- und materialaufwendig und führt zu einem signifikanten Verlust an Aldehyd, da die zur vollständigen Gewinnung des hochsiedenden Aldehyds erforderlichen hohen Temperaturen die Bildung von hochsiedenden Nebenprodukten verstärken.

Mit dem in der EP 484 977 A beschriebenen Verfahren wurde versucht, diese Nachteile zu vermeiden. Das Verfahren besteht darin, dass man für die Auftrennung des Rohaldehydgemisches nur eine Destillationskolonne verwendet und die Destillationsbedingungen so wählt, dass die verzweigten Aldehyde in flüssiger Form im oberen Bereich der Destillationskolonne abgenommen werden und der geradkettige Aldehyd in zwei Produktströme zerlegt wird. Der erste Produktstrom, bei dem es sich um im Wesentlichen reinen, geradkettigen Aldehyd handelt, wird dampfförmig im unteren Bereich der Destillationskolonne abgenommen. Die Menge dieses Aldehydproduktstromes beträgt nicht mehr als 70 Gew.-% der gesamten Menge an geradkettigem Aldehyd im Rohaldehydgemisch. Der zweite Produktstrom aus geradkettigem Aldehyd wird am Sumpf abgenommen, er enthält die Hauptmenge der hochsiedenden Bestandteile. Das Verfahren ermöglicht zwar die Abtrennung der verzweigten Aldehyde, die geradkettigen Aldehyde.aber werden lediglich in zwei Fraktionen unterschiedlicher Reinheit aufgetrennt, wobei die Menge der ersten relativ reinen Fraktion max. 70 % des eingesetzten Produktes beträgt, um die Menge an hochsiedenden Bestandteilen in der zweiten Fraktion nicht allzu hoch ansteigen zu lassen. Die mit diesem Verfahren in der Praxis erzielbare Menge an reinem geradkettigem Aldehyd liegt somit lediglich bei max. 70 Gew.-% des im Rohproduktgemisch vorhandenen geradkettigen Aldehyds.

Der vorliegenden Erfindung liegt daher die Aufgabe zugrunde, ein verfahren zur Auftrennung eines flüssigen Rohaldehydgemisches zur Verfügung zu stellen, das weniger aufwendig ist und es erlaubt, den geradkettigen und den verzweigten Aldehyd möglichst vollständig in reiner Form zu gewinnen.

Überraschenderweise wurde nun gefunden, dass diese Aufgabe gelöst wird, wenn man in einer ersten Destillationskolonne das Rohaldehydgemisch so auftrennt, dass der verzweigte Aldehyd über oder nahe dem Kopf der Destillationskolonne abgenommen wird, der geradkettige Aldehyd im unteren Bereich der Kolonne als Seitenstrom abgenommen wird und ein weiterer Produktstrom, der den restlichen geradkettigen Aldehyd und die hochsiedenden Bestandteile enthält, über Sumpf abgenommen und in einer zweiten, kleineren Destillationskolonne aufgetrennt wird.

Gegenstand der vorliegenden Erfindung ist daher ein Verfahren zur destillativen Auftrennung eines Rohaldehydgemisches, das im Wesentlichen 94 bis 99,8 Gew.-%, bezogen auf das Gesamtgewicht des Rohaldehydgemisches, eines geradkettigen und einen oder mehrere verzweigte Aldehyde (geradkettige und verzweigte Alkohole weisen die gleiche Kohlenstoffzahl auf) enthält, wobei das Verfahren dadurch gekennzeichnet ist, dass man
A) das Rohaldehydgemisch in den mittleren Bereich einer ersten Destillationskolonne mit mehreren Trennstufen einspeist und darin eine Auftrennung vornimmt in
   i) einen ersten Aldehydproduktstrom, der über oder nahe am Kopf des Destillationskolonne abgenommen wird und im Wesentlichen reinen verzweigten Aldehyd umfasst,
   ii) einen zweiten Aldehydproduktstrom, der direkt oberhalb des Verdampfers oder darüber im Bereich der ersten 20 % der Gesamttrennstufen abgenommen wird und der im Wesentlichen reinen geradkettigen Aldehyd umfasst, und
   iii)einen weiteren Produktstrom, der die hochsiedenden Bestandteile und 75 bis 93 Gew.-%, bezogen auf das Gesamtgewicht des weiteren Produktstroms, geradkettige Aldehyde umfasst, und
B) den weiteren Produktstrom am Sumpf der ersten Destillationskolonne abnimmt und in eine zweite Destillationskolonne leitet, deren Packungsvolumen um den Faktor 50 bis 200 kleiner ist als das der ersten Destillationskolonne, und darin in einen Produktstrom, der im Wesentlichen reinen geradkettigen Aldehyd umfasst und über Kopf oder nahe am Kopf der zweiten Destillationskolonne abgenommen wird, und in einen Produktstrom, der im Wesentlichen die hochsiedenden Bestandteile umfasst, aufgetrennt wird.

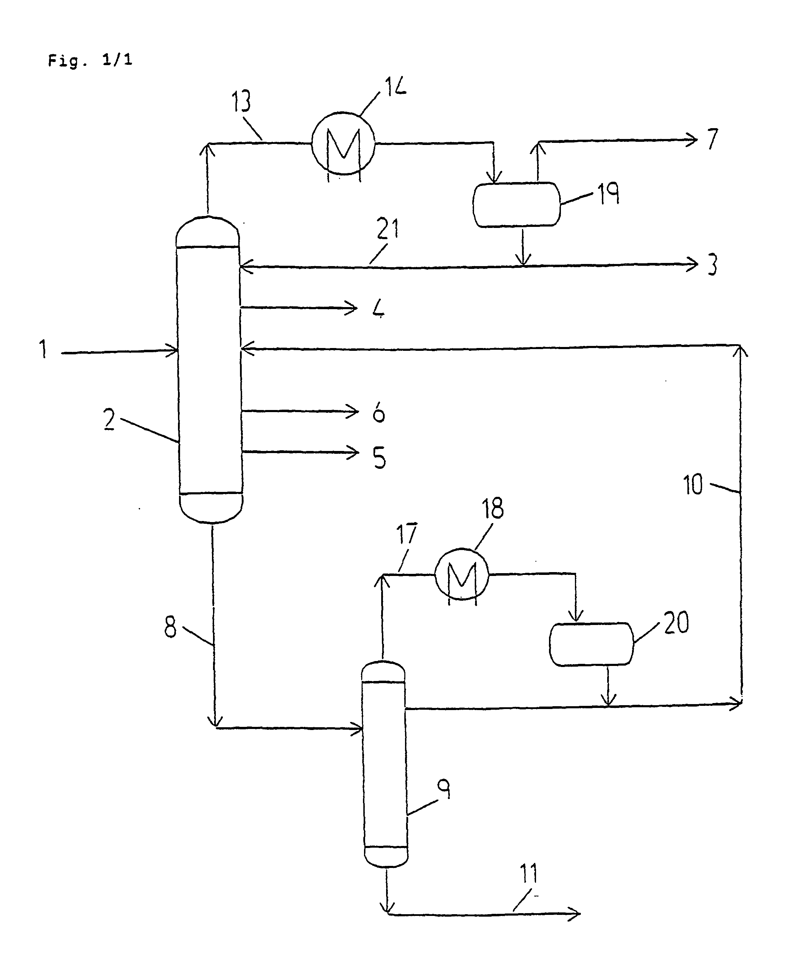

Die Figur 1 zeigt eine schematische Darstellung des erfindungsgemäßen Verfahrens.

Als Rohaldehydgemisch kann jedes Produktgemisch eingesetzt werden, das aus Hydroformylierungsverfahren erhalten wird. Bei der Hydroformylierung handelt es sich um ein bekanntes Verfahren, das beispielsweise in den US-Patenten 4,148,830; 4,247,486; 4,593,127 und in der EP 404 193 und EP 484 977 A beschrieben ist.

Als Ausgangsmaterial für die Hydroformylierung verwendet man insbesondere Olefine mit drei oder vier Kohlenstoffatomen, wie Propylen, 1-Buten, 2-Buten (cis oder trans) und Isobutylen. Das erfindungsgemäß aufzutrennende Rohaldehydgemisch enthält daher vorzugsweise geradkettige und verzweigte C₄- oder C₅-Aldehyde.

Die für die Hydroformylierung verwendeten Katalysatoren sind ebenfalls bekannt, sie sind beispielsweise in den in der EP 484 977 A zitierten Patentpublikationen beschrieben.

Das bei der Hydroformylierung anfallende Rohaldehydgemisch enthält neben leichter siedenden Bestandteilen, wie Kohlenmonoxid und Wasserstoff, verzweigte Aldehyde, geradkettige Aldehyde und höher siedende Bestandteile. Letztere sind Nebenprodukte, die durch Kondensation der Aldehyde zu Dimeren, Trimeren und Tetrameren und durch Reduktion der entstandenen Aldehyde mit Wasserstoff zu den entsprechenden Alkoholen entstanden sind. Als Aldehyde kann das Rohaldehydgemisch, insbesondere die durch Hydroformylierung von Propylen erhaltenen C₄-Aldehyde, wie n-Butyraldehyd und Isobutyraldehyd, oder die durch Hydroformylierung von Buten erhaltenen C₅-Aldehyde, wie n-Valeraldehyd, und die verzweigten C₅-Aldehyde, 2-Methylbutyraldehyd, 3-Methylbutyraldehyd und Pivaldehyd, enthalten. Die Zusammensetzung der Rohaldehydgemische ist abhängig von den Bedingungen des Hydroformylierungsprozesses. Das gewichtsverhältnis von geradkettigem Aldehyd zu verzweigtem(n) Aldehyd(en) liegt im Allgemeinen im Bereich von etwa 8:2 bis etwa 99:1. Die Gesamtmenge an Aldehyden im Rohaldehydgemisch beträgt im Allgemeinen 94 bis 99,8 Gew.-%, vorzugsweise 97 bis 99,8 Gew.-%. Den Rest bilden die oben erwähnten leichter und insbesondere die höher siedenden Bestandteile, nämlich jeweils etwa 0,1 bis 3 Gew.-% der Kondensationsprodukte und der Alkohole.

Das erfindungsgemäße Verfahren kann in jeder geeigneten Destillationskolonne durchgeführt werden. Geeignet sind beispielsweise Bodenkolonnen, wie Ventilbodenkolonnen, und vorzugsweise Kolonnen mit Packungen. Bei den Packungen kann es sich um übliche regellose Schüttungen oder um geordnete Packungen handeln, wobei letztere bevorzugt sind. Insbesondere verwendet man als Packung Mellapak® der Fa. Sulzer (geordnete Packung).

Die Zahl der theoretischen Trennstufen muss so groß sein, dass die gewünschte Auftrennung bewirkt wird. Sie liegt im Allgemeinen im Bereich von etwa 40 bis 100 theoretischen Trennstufen.

Die erste Destillationskolonne ist mit den für den Betrieb erforderlichen Mitteln, wie Verdampfer, Kondensator, Mess- und Regeleinrichtungen, Anschlüssen für die Zufuhr und Abnahme der Produkte etc. ausgestattet. Das Rohaldehydgemisch wird im Allgemeinen in den mittleren Bereich (etwa das mittlere Drittel) der Destillationskolonne, bezogen auf die Zahl der theoretischen Trennstufen, kontinuierlich eingespeist. Der genaue Punkt, an dem das Gemisch eingespeist wird, ist nicht kritisch, er kann vom Fachmann nach Standardmethoden bestimmt werden.

Die Destillationsbedingungen in der ersten Destillationskolonne werden so gewählt, dass man eine Auftrennung in die nachfolgend erläuterten Produktströme erzielt. Die Sumpftemperatur liegt dabei im Allgemeinen etwa 1 bis 40 °C, vorzugsweise etwa 10 bis 30 °C, oberhalb des Siedepunktes des geradkettigen Aldehyds. Der Druck im Kopf der Kolonne liegt im Allgemeinen im Bereich von 1,0 bar bis 1,5 bar (absolut).

Unter diesen Umständen erfolgt in der ersten Destillationskolonne eine Auftrennung in die drei folgenden Produktströme:
i) Einen ersten Aldehydproduktstrom, der über Kopf oder nahe am Kopf der Destillationskolonne als flüssiger Seitenabzug abgenommen wird. Es handelt sich dabei um im Wesentlichen reinen verzweigten Aldehyd. Die über Kopf destillierten verzweigten Aldehyde werden in üblicher Weise kondensiert, wobei ein Teil des Kondensates als Rücklauf in die Kolonne zurückgeführt werden kann. Das Rücklaufverhältnis liegt im Allgemeinen im Bereich von 20 bis 30. Der reine verzweigte Aldehyd verlässt dann die Anlage. Soweit in dem Rohaldehydgemisch noch leichter flüchtige Bestandteile als die verzweigten Aldehyde vorhanden sind, beispielsweise Kohlenmonoxid, Wasserstoff, Olefine und Paraffine, werden diese ebenfalls über Kopf abgenommen und ausgeschleust.
ii) Einen zweiten Aldehydproduktstrom, der direkt oberhalb des Verdampfers oder darüber im Bereich der ersten 20 % der Gesamttrennstufen abgenommen wird. Vorzugsweise wird der zweite Aldehydproduktstrom im Bereich der ersten 10 % der Gesamtzahl der theoretischen Trennstufen, insbesondere unterhalb der ersten Trennstufe, d. h. direkt über dem Sumpf, abgenommen. Besonders bevorzugt wird der Aldehydproduktstrom dampfförmig abgenommen.
   Bei dem zweiten Aldehydproduktstrom handelt es sich um im Wesentlichen reinen geradkettigen Aldehyd. Im Allgemeinen werden mehr als 70 Gew.-%, vorzugsweise mehr als 80 Gew.-%, insbesondere mehr als 90 Gew.-% des gesamten, im Rohaldehydgemisch vorhandenen geradkettigen Aldehyds abgenommen.
iii) Einen weiteren, hochsiedenden Produktstrom, der am Sumpf der Kolonne flüssig abgenommen wird. Dieser Produktstrom enthält die oben erwähnten hochsiedenden Bestandteile und 75 bis 93 Gew.-%, vorzugsweise 80 bis 93 Gew.-%, insbesondere 85 bis 93 Gew.-% und besonders bevorzugt 88 bis 92 Gew.-%, bezogen auf das Gesamtgewicht des weiteren hochsiedenden Produktstromes, an geradkettigem Aldehyd. Die Menge an geradkettigem Aldehyd entspricht im Allgemeinen etwa 2 bis 5 Gew.-% der Gesamtmenge an geradkettigem Aldehyd.

Der weitere hochsiedende Produktstrom iii) wird dann in den mittleren Bereich einer zweiten (etwa das mittlere Drittel der theoretischen Trennstufen) Destillationskolonne eingespeist. Diese ist kleiner als die erste Destillationskolonne. Der Unterschied lässt sich durch das Packungsvolumen ausdrücken, d. h. das Pakkungsvolumen der zweiten Kolonne ist um den Faktor 50 bis 200, vorzugsweise 80 bis 180, insbesondere 100 bis 170 und besonders bevorzugt 120 bis 160 kleiner als das Packungsvolumen der ersten Kolonne.

Das Verhältnis des Durchmessers von erster zu zweiter Kolonne liegt im Bereich von 10:1 bis 4:1.

Die Kolonne ist mit den für den Betrieb erforderlichen Mitteln, wie Verdampfer, Kondensator, Mess- und Regeleinrichtungen, Anschlüssen für die Zufuhr und Abnahme der Produkte etc., ausgestattet.

Die Destillationsbedingungen werden so gewählt, dass man eine Auftrennung in einen Produktstrom, der im Wesentlichen reinen geradkettigen Aldehyd umfasst, und einen Produktstrom, der die Hochsieder umfasst, erhält. Die Sumpftemperatur liegt dabei im Allgemeinen etwa 1 bis 40 °C, vorzugsweise etwa 10 bis 30 °C, oberhalb des Siedepunktes des geradkettigen Aldehyds. Der Druck im Kopf der Kolonne liegt im Allgemeinen im Bereich von 1,0 bar bis 1,5 bar (absolut). Der reine geradkettige Aldehyd wird über Kopf oder nahe am Kopf der Destillationskolonne, vorzugsweise flüssig, abgenommen und besonders bevorzugt wieder in die erste Destillationskolonne oberhalb der Stelle, an welcher der Produktstrom ii) abgenommen wird, eingespeist. Vorzugsweise erfolgt die Einspeisung etwa in gleicher Höhe wie die Einspeisung des Rohaldehydgemisches, z. B. durch Zumischen in den Zulauf des Rohaldehydgemisches. Bei dieser bevorzugten Ausführungsform fällt im Wesentlichen der gesamte, im Rohaldehydgemisch vorhandene geradkettige Aldehyd als Produktstrom ii) an. Der die Hochsieder enthaltende Produktstrom wird über den Sumpf der zweiten Destillationskolonne abgezogen und verlässt die Anlage.

Das erfindungsgemäße Verfahren hat den Vorteil, dass es mit geringerem Aufwand und unter schonenden Bedingungen durchgeführt werden kann und im Wesentlichen die gesamten geradkettigen und verzweigten Aldehyde in reiner Form erhalten werden. Sowohl die geradkettigen als auch die verzweigten Aldehyde werden in einer Reinheit von 99 bis 99,98 Gew.-% erhalten. Im Allgemeinen beträgt der Hochsiedergehalt in den geradkettigen Aldehyden weniger'als 0,5 Gew.-%, insbesondere weniger als 0,2 Gew.-%.

Die nachfolgenden Beispiele erläutern unter Bezug auf die Figur die Erfindung, ohne sie zu beschränken.

### Beispiel 1

Als Ausgangsmaterial wurde ein aus der Hydroformylierung stammendes Rohaldehydgemisch aus Isobutyraldehyd und n-Butyraldehyd mit einem Anteil von 0,01 Gew.-% leichter siedenden Bestandteilen als Isobutyraldehyd und 0,5 Gew.-% höher siedenden Bestandteilen als n-Butyraldehyd verwendet. Als erste Destillationskolonne wurde eine Kolonne 2 mit 80 theoretischen Trennstufen verwendet, die mit Mellapak® der Fa. Sulzer (geordnete Packung) gepackt war. Das Rohaldehydgemisch wurde als Zulauf 1 mit einer Masse von 10 000 kg/h auf die 43. Trennstufe der Kolonne 2 aufgegeben. Die Destillation erfolgte bei einer Sumpftemperatur von 87 °C und bei einem absoluten Kopfdruck von 1,24 bar. Isobutyraldehyd sowie Leichtsieder wurden über Kopf als Strom 13 abgenommen. Der Isobutyraldehyd wurde im Kondensator 14 kondensiert und im Behälter 19 aufgefangen. Die leichtflüchtigen, nichtkondensierbaren Anteile wurden als Strom 7 aus der Anlage ausgeschleust. Ein Teil des im Behälter 19 aufgefangenen Kondensats wurde als Rücklaufstrom 21 in den Kopfbereich der Kolonne 2 zurückgeführt (Rücklaufverhältnis 22). Reiner Isobutyraldehyd wurde als Strom 3 in einer Menge von ca. 1 400 kg/h aus der Anlage ausgeschleust.

Reiner n-Butyraldehyd wurde als dampfförmiger Seitenstrom 5 in einer Menge von ca. 8 600 kg/h unterhalb der ersten Trennstufe der Kolonne 2 entnommen. Der hochsiedende Produktstrom 8 wurde als Sumpfabzug von ca. 600 kg/h in die zweite Destillationskolonne 9 geleitet, die ebenfalls mit Mellapak® der Fa. Sulzer gepackt war. Ihr Packungsvolumen war etwa um den Faktor 140 kleiner als das der ersten Destillationskolonne (Durchmesserverhältnis von großer zu kleiner Kolonne etwa 8). Die Destillation in der zweiten Kolonne wurde bei einer Sumpftemperatur von 134 °C und bei einem absoluten Kopfdruck von 1,2 bar durchgeführt. Auf diese Weise erfolgte eine Auftrennung in ca. 550 kg/h n-Butyraldehyd, der als Strom 10 nahe am Kopf der Kolonne 9 abgenommen und auf die 43. Trennstufe der Kolonne 2 zurückgeführt wurde. Alternativ kann der n-Butyraldehyd als Strom 17 über Kopf abgenommen, im Kondensator 18 kondensiert und im Behälter 20 aufgefangen und gegebenenfalls in die Kolonne 2 zurückgeführt werden. Der Sumpfabzug der Kolonne 9 wurde als Strom 11 in einer Menge von etwa 50 kg/h aus der Anlage ausgeschleust.

Die Reinheit des erhaltenen Isobutyraldehyds war 99,9 Gew.-% und die Reinheit des n-Butyraldehyds war 99,75 Gew.-%. Die im Zulauf 1 enthaltenen Aldehyde wurden zu 99,9 % als Reinkomponenten gewonnen.

### Beispiel 2

Das folgende Beispiel einer Computersimulation dient zur weiteren Veranschaulichung der Erfindung, wobei ebenfalls auf die Figur Bezug genommen wird. Ein Rohaldehydgemisch aus Isobutyraldehyd und n-Butyraldehyd mit einem Anteil von 0,01 Gew.-% leichter siedenden Bestandteilen als Isobutyraldehyd und etwa 1,3 Gew.-% höher siedenden Bestandteilen als n-Butyraldehyd wird mit einer Masse von 9 500 kg/h als Zulauf 1 auf die 58. Trennstufe der Kolonne 2 (gleiche Kolonne wie in Beispiel 1) aufgegeben. Die Destillation erfolgt bei einer Sumpftemperatur der Kolonne 2 von 98 °C und bei einem absoluten Kopfdruck von 1,2 bar. Isobutyraldehyd sowie die Leichtsieder werden über Kopf als Strom 13 abgenommen und im Kondensator 14 bis auf die gasförmigen Anteile, welche als Strom 7 die Anlage verlassen (1 kg/h), kondensiert. Ein Teil des im Behälter 19 aufgefangenen Kondensats wird als Rücklauf 21 in die Kolonne 2 zurückgegeben (Rücklaufverhältnis 30), während reiner Isobutyraldehyd (etwa 1 400 kg/h) als Strom 3 ausgeschleust wird. Ein dampfförmiger Seitenstrom 6 aus etwa 7 974 kg/h reinem n-Butyraldehyd wurde unterhalb der 10. Trennstufe der Kolonne 2 entnommen. Der flüssige Sumpfabzug 8 von ca. 600 kg/h wird in den mittleren Bereich der Kolonne 9 (gleiche Kolonne wie im Beispiel 1) eingespeist. Die Destillation in der Kolonne 9 erfolgt bei 134 °C Sumpftemperatur und bei einem Kopfdruck von 1,2 bar (absolut). Es werden 475 kg/h n-Butyraldehyd als Strom 10 im Kopfbereich der Kolonne abgenommen und in den mittleren Bereich der Kolonne 2 zurückgeführt. Der Sumpfabzug 11 der Kolonne 9 von etwa 125 kg/h enthält die höher siedenden Bestandteile und verlässt die Anlage. Die Reinheit des gewonnenen n- und Isobutyraldehyds beträgt jeweils 99,9 Gew.-%.

### Beispiel 3

Auch die folgende Computersimulation dient zur weiteren Veranschaulichung der Erfindung unter Bezug auf die Figur.

Ein Rohaldehydgemisch aus Iso-Valeraldehyd und n-Valeraldehyd mit einem Anteil von 0,2 Gew.-% leichter siedenden Bestandteilen als Isovaleraldehyd und etwa 0,6 Gew.-% höher siedenden Bestandteilen als n-Valeraldehyd wird mit einer Masse von 11 000 kg/h als Zulauf 1 auf die 58. Trennstufe der Kolonne (gleiche Kolonne wie in Beispiel 1) aufgegeben. Die Destillation erfolgt bei einer Sumpftemperatur der Kolonne von 120 °C und bei einem absoluten Kopfdruck von 1,2 bar. Isovaleraldehyd sowie Leichtsieder werden über Kopf als Strom 13 gewonnen und im Kondensator 14 bis auf die gasförmigen Anteile, welche als Strom 7 die Anlage verlassen (1 kg/h), kondensiert. Ein Teil des im Behälter 19 aufgefangenen Kondensats wird als Rücklauf 21 (Rücklauf Verhältnis 30) in die Kolonne 2 zurückgeführt, während reiner Isovaleraldehyd (etwa 1 650 kg/h) als Strom 3 die Anlage verlässt. Reiner n-Valeraldehyd wird als dampfförmiger Seitenstrom 6 in einer Menge von etwa 9 279 kg/h unterhalb der 10. Trennstufe der Kolonne 2 entnommen. Der flüssige Sumpfabzug 8 von etwa 600 kg/h wird in den mittleren Bereich der Kolonne 9 (gleiche Kolonne wie im Beispiel 1) geleitet. Die Destillation in der Kolonne 9 erfolgt bei einer Sumpftemperatur von 170 °C und bei einem Kopfdruck von 1,2 bar (absolut). Der Sumpfabzug 11 dieser Kolonne enthält mit etwa 70 kg/h die höher siedenden Bestandteile, welche die Anlage verlassen. Über Kopf der Kolonne 9 werden etwa 530 kg/h n-Valeraldehyd als Strom 10 abgenommen und in den mittleren Bereich der Kolonne 2 zurückgegeben. Die Reinheit des erhaltenen n- und Isovaleraldehyds beträgt jeweils 99,9 Gew.-%.

## Patentansprüche

1. Verfahren zur destillativen Auftrennung eines Rohaldehydgemisches, das im Wesentlichen 94 bis 99,8 Gew.-%, bezogen auf das Gesamtgewicht des Rohaldehydgemisches, eines geradkettigen und mindestens eines verzweigten Aldehyds enthält, **dadurch gekennzeichnet, dass** man
A) das Rohaldehydgemisch in den mittleren Bereich einer ersten Destillationskolonne mit mehreren Trennstufen einspeist und darin eine Auftrennung vornimmt in
i) einen ersten Aldehydproduktstrom, der über oder nahe dem Kopf der Destillationskolonne abgenommen wird und im Wesentlichen reinen verzweigten Aldehyd umfasst,
ii) einen zweiten Aldehydproduktstrom, der direkt oberhalb des Verdampfers oder darüber im Bereich der ersten 20 % der Gesamttrennstufen abgenommen wird und der im Wesentlichen reinen geradkettigen Aldehyd umfasst, und
iii) einen weiteren Produktstrom, der Hochsieder und 75 bis 93 Gew.-%, bezogen auf das Gesamtgewicht des weiteren Produktstroms, geradkettigen Aldehyd umfasst und
B) den weiteren Produktstrom in eine zweite Destillationskolonne leitet, deren Packungsvolumen um den Faktor 50 bis 200 kleiner ist als das der ersten Destillationskolonne und darin in einen Aldehydproduktstrom, der im Wesentlichen gereinigten geradkettigen Aldehyd umfasst und über Kopf oder nahe am Kopf der Destillationskolonne abgenommen wird, und in einen Produktstrom, der höher als der geradkettige Aldehyd siedende Bestandteile umfasst, aufgetrennt wird.

2. Verfahren nach Anspruch 1, **dadurch gekennzeichnet, dass** der Aldehydproduktstrom ii) dampfförmig abgenommen wird.

3. Verfahren nach Anspruch 1 oder 2, **dadurch gekennzeichnet, dass** der Aldehydproduktstrom ii) im Bereich der ersten 10 Trennstufen der ersten Destillationskolonne abgenommen wird.

4. Verfahren nach einem der vorhergehenden Ansprüche, **dadurch gekennzeichnet, dass** mehr als 70 Gew.-% des gesamten, im Rohaldehydgemisch enthaltenen geradkettigen Alkohols als Produktstrom ii) gewonnen werden.

5. Verfahren nach Anspruch 4, **dadurch gekennzeichnet, dass** mehr als 90 Gew.-% des gesamten, im Rohaldehydgemisch enthaltenen geradkettigen Alkohols als Produktstrom ii) gewonnen werden.

6. Verfahren nach einem der vorhergehenden Ansprüche, **dadurch gekennzeichnet, dass** der weitere Produktstrom iii) 75 bis 92 Gew.-% geradkettigen Aldehyd enthält.

7. Verfahren nach einem der vorhergehenden Ansprüche, **dadurch gekennzeichnet, dass** der in der zweiten Destillationskolonne gewonnene Aldehydproduktstrom in die erste Destillationskolonne zurückgeführt wird.

8. Verfahren nach einem der vorhergehenden Ansprüche, **dadurch gekennzeichnet, dass** das Rohaldehydgemisch geradkettige und verzweigte C₄- oder C₅-Aldehyde enthält.

## Claims

1. A process for the fractional distillation of a crude aldehyde mixture comprising essentially from 94 to 99.8% by weight, based on the total weight of the crude aldehyde mixture, of a straight-chain and at least one branched aldehyde, which comprises
A) feeding the crude aldehyde mixture into the middle region of a first distillation column having a plurality of theoretical plates and fractionating it in the column into
i) a first aldehyde product stream which is taken off at or near the top of the distillation column and comprises essentially pure branched aldehyde,
ii) a second aldehyde product stream which is taken off immediately above the vaporizer or further up in the region of the first 20% of the total theoretical plates and comprises essentially pure straight-chain aldehyde, and
iii) a further product stream which comprises the high-boiling constituents and from 75 to 93% by weight, based on the total weight of the further product stream, of straight-chain aldehydes, and
B) passing the further product stream to a second distillation column whose packing volume is a factor of from 50 to 200 smaller than that of the first distillation column, and, in this second distillation column, fractionating it into an aldehyde product stream which comprises essentially purified straight-chain aldehyde and is taken off at the top or near the top of the second distillation column and a product stream which comprises constituents having a boiling point higher than that of the straight-chain aldehyde.

2. A process as claimed in claim 1, wherein the aldehyde product stream ii) is taken off in vapor form.

3. A process as claimed in claim 1 or 2, wherein the aldehyde product stream ii) is taken off in the region of the first 10 theoretical plates of the first distillation column.

4. A process as claimed in any of the preceding claims, wherein more than 70% by weight of the total straight-chain alcohol present in the crude aldehyde mixture is obtained as product stream ii).

5. A process as claimed in claim 4, wherein more than 90% by weight of the total straight-chain alcohol present in the crude aldehyde mixture is obtained as product stream ii).

6. A process as claimed in any of the preceding claims, wherein the further product stream iii) contains from 75 to 92% by weight of straight-chain aldehyde.

7. A process as claimed in any of the preceding claims, wherein the aldehyde product stream obtained in the second distillation column is returned to the first distillation column.

8. A process as claimed in any of the preceding claims, wherein the crude aldehyde mixture comprises straight-chain and branched C₄- or C₅-aldehydes.

## Revendications

1. Procédé de rectification d'un mélange d'aldéhydes brut qui contient essentiellement 94% à 99,8% en poids, par rapport au poids total du mélange d'aldéhydes brut, d'un aldéhyde à chaîne droite et d'au moins un aldéhyde ramifié, **caractérisé en ce que**
A) l'on conduit le mélange d'aldéhydes brut dans la zone centrale d'une première colonne de distillation comprenant plusieurs étages de séparation, et on y réalise une séparation en
i) un premier courant de produit d'aldéhyde qui est soutiré au niveau ou près de la tête de la colonne de distillation et qui comprend principalement un aldéhyde ramifié pur,
ii) un deuxième courant de produit d'aldéhyde, qui est soutiré directement au niveau de l'évaporateur ou au-dessus de celui-ci dans la zone des premiers 20% de la totalité des étages de séparation, et qui comprend principalement un aldéhyde à chaîne droite pur, et
iii) un autre courant de produit qui comprend des produits à point d'ébullition élevé et 75% à 93%, par rapport au poids total de l'autre courant de produit, d'un aldéhyde à chaîne droite et
B) l'on conduit l'autre courant de produit dans une deuxième colonne de distillation dont le volume de remplissage est 50 à 200 fois inférieur à celui de la première colonne de distillation, et on y réalise une séparation en un courant de produit d'aldéhyde, qui comprend principalement un aldéhyde à chaîne droite purifié et qui est soutiré au niveau de la tête ou près de la tête de la colonne de distillation, et en un courant de produit qui comprend des composants à point d'ébullition plus élevé que l'aldéhyde à chaîne droite.

2. Procédé selon la revendication 1, **caractérisé en ce que** le courant de produit d'aldéhyde ii) est soutiré à l'état de vapeur.

3. Procédé selon la revendication 1 ou 2, **caractérisé en ce que** le courant de produit d'aldéhyde ii) est soutiré dans la zone des premiers 10 étages de la première colonne de distillation.

4. Procédé selon l'une quelconque des revendications précédentes, **caractérisé en ce que** l'on obtient plus de 70% en poids de la totalité de l'alcool à chaîne droite contenu dans le mélange d'aldéhydes brut en tant que courant de produit ii).

5. Procédé selon la revendication 4, **caractérisé en ce que** l'on obtient plus de 90% en poids de la totalité de l'alcool à chaîne droite contenu dans le mélange d'aldéhydes brut en tant que courant de produit ii).

6. Procédé selon l'une quelconque des revendications précédentes, **caractérisé en ce que** l'autre courant de produit iii) contient de 75% à 92% en poids d'aldéhyde à chaîne droite.

7. Procédé selon l'une quelconque des revendications précédentes, **caractérisé en ce que** le courant de produit d'aldéhyde obtenu dans la deuxième colonne de distillation est recyclé dans la première colonne de distillation

8. Procédé selon l'une quelconque des revendications précédentes, **caractérisé en ce que** le mélange d'aldéhydes brut contient des aldéhydes en C₄ ou en C₅ à chaîne droite ou ramifiés.
